# EUROPÄISCHE PATENTANMELDUNG

(11) **EP 0 963 052 A2**
(43) Veröffentlichungstag der Anmeldung: **08.12.1999**
(21) Anmeldenummer: 99109775.9
(22) Anmeldetag: 18.05.1999
(51) Int. Cl.: H04B 1/20, G10K 15/00, G11B 20/00, A61M 21/00, B60R 11/02

(54) **Vorrichtung zur Beeinflüssung eines Audiosignals in einem Kraftfahrzeug**

(30) Priorität: 05.06.1998 DE 19825280
(71) Anmelder: Bayerische Motoren Werke Aktiengesellschaft, 80788 München (DE)
(72) Erfinder: Beer, Rainer, 80937 München (DE); Herrmann, Friedrich, Alias Glorian, Friedrich, 89077 Ulm (DE)
(74) Vertreter: Wesel-Mair, Julia (DE)

(57) **Zusammenfassung**

Bei einer Vorrichtung zur Beeinflussung eines Audiosignais in einem Kraftfahrzeug mit einem Hüllkurvenmodulator moduliert der Hüllkurvenmodulator das Audiosignal mit einer Hüllkurve, die eine die Wachheit des Fahrzeuginsassen beeinflussende Frequenz aufweist.

Vorzugsweise ist eine manuelle Auswahleinheit vorgesehen, durch die mindestens eine Hüllkurve mit einer die Wachheit fördernden Frequenz und mindestens eine Hüllkurve mit einer der Wachheit entgegenwirkenden (entspannenden) Frequenz auswählbar ist.

Zusätzlich oder alternativ ist eine automatische Auswahleinheit vorgesehen, durch die in Abhängigkeit von mindestens einem Fahrzeugparameter (v) mindestens eine Hüllkurve mit einer die Wachheit fördernden Frequenz und mindestens eine Hüllkurve mit einer der Wachheit entgegenwirkenden (entspannenden) Frequenz auswählbar ist.

## Beschreibung

Die Erfindung bezieht sich auf eine Vorrichtung zur Beeinflussung eines Audiosignals in einem Kraftfahrzeug.

Es sind bereits Vorrichtungen zur Beeinflussung eines Audiosignals in einem Kraftfahrzeug beispielsweise in Form von digitalen Signalprozessoren (DSP) bekannt, womit Audiosignale beliebig in ihren Eigenschaften verändert werden können. Diese Eigenschaften werden genutzt, um insbesondere unvorteilhafte klangliche Eigenschaften der Peripherie, in der sich die Audioanlage befindet, auszugleichen. Zusätzlich können klangliche Eigenschaften am Audiosignal derart verändert werden, daß sie den persönlichen Geschmack des Kunden zufriedenstellen. Dabei werden vorzugsweise die Pegel bestimmter Frequenzbereiche gegenüber anderen Frequenzbereichen erhöht oder reduziert.

Weiterhin sind therapeutische Vorrichtungen, wie z. B. "brain machines" oder Klangschalen, bekannt, die durch akustische Effekte eine Entspannung des Anwenders bewirken sollen. Bei der Untersuchung der Akustik von Klangschalen im Zusammenhang mit ihrem Einsatz in der Therapie hat sich herausgestellt, daß Schwingungen ca. zwischen 13 und 20 Hz die Wachheit des Zuhörers, dagegen Schwingungen zwischen ca. 8 und 13 Hz die Entspannung eines Zuhörers fördern. Diese Erkenntnis wird bereits genutzt, um die Hüllkurven von für die Klangtherapie ausgewählten Musikstücken manuell mittels eines Mischpultes entsprechend zu modulieren. Bei diesem bekannten Verfahren ist es jedoch erforderlich, das zu modulierende Musikstück vorher zu kennen.

Es ist Aufgabe der Erfindung, eine Vorrichtung zur Beeinflussung eines Audiosignals in einem Kraftfahrzeug zu schaffen, durch die die Wachheit oder ggf. die Entspannung eines Fahrzeuginsassen in einem Kraftfahrzeug gefördert wird.

Diese Aufgabe wird durch die Merkmale des Patentanspruchs 1 gelöst. Vorteilhafte Weiterbildungen der Erfindung sind die Gegenstände der abhängigen Patentansprüche.

Die Erfindung besteht im wesentlichen in der Verwendung der aus der Klangtherapie bekannten Erkenntnisse in Kraftfahrzeugen. Da jedoch die Audiosignale in einem Kraftfahrzeug vor deren Beeinflussung im Unterschied zum bekannten Verfahren aus der Klangtherapie nicht bekannt bzw. vorhersagbar sind, findet erfindungsgemäß eine automatische Hüllkurvenmodulation des Audiosignals über alle Audiosignale mit einer Frequenz statt, die die Wachheit des Fahrzeuginsassen, insbesondere aus empirisch ermittelten Erfahrungswerten, in gewünschter Weise beeinflussen.

Bei der erfindungsgemäßen Hüllkurvenmodulation von Audiosignalen in einem Kraftfahrzeug ist insbesondere darauf zu achten, daß der Modulationshub, d. h. der Pegel der Hüllkurvenschwingung, nur so hoch ist, daß das Audiosignal nicht hörbar verfälscht wird, um keine störenden akustischen Nachteile zu erhalten. Dabei ist es entweder möglich, mit einem konstanten Modulationshub oder einem variablen Modulationshub zu arbeiten, der dem aktuell auftretenden Audiosignal in Echtzeit angepaßt wird, um andererseits einen maximalen Effekt zu erhalten.

Wird eine erfindungsgemäße Vorrichtung mit lediglich einer nicht verstellbaren Modulationsfrequenz gewählt, ist darauf zu achten, daß im Gegensatz zur Anwendung in der Klangtherapie nicht die Entspannung sondern die Wachheit des Fahrzeuginsassen, insbesondere des Fahrers, gefördert wird.

Beispielsweise kann jedoch auch manuell oder automatisch zwischen mindestens zwei Modulationsfrequenzen umgeschaltet werden, die entweder die Wachheit oder die Entspannung fördern. Bei einer automatischen Umschaltung der Modulationsfrequenz wird diese insbesondere in Abhängigkeit vom Fahrzustand, d. h. in Abhängigkeit von mindestens einem Fahrzeugparameter, vorgenommen.

Vorzugsweise wird der Modulationshub in Abhängigkeit von der Bandbreite des Audiosignals eingestellt. Diese Anpassung des Modulationshubs an die Bandbreite des Audiosignals kann beispielsweise kontinuierlich oder aber in Form von Kategorien stufenweise vorgenommen werden.

Mit dieser erfindungsgemäßen Vorrichtung findet eine auf die Erfordernisse in Kraftfahrzeugen angepaßte Verwendung von aus der Klangtherapie bekannten Erkenntnisse statt.

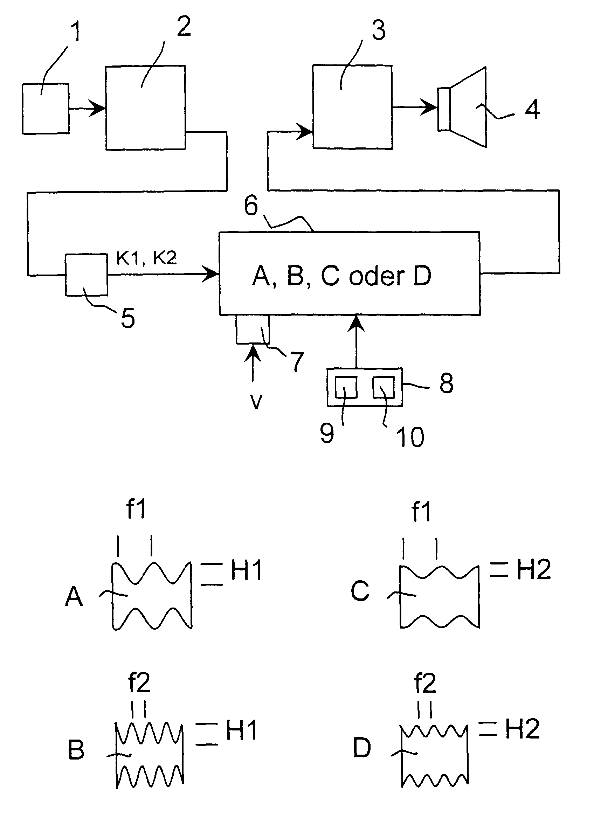

In der Zeichnung ist ein Ausführungsbeispiel der Erfindung dargestellt. Sie zeigt eine Vorrichtung zur Beeinflussung eines Audiosignals in Form eines Radiosignals.

Ein Audiosignal wird vom Ausgang eines Radios 1 zunächst an eine manuelle Klangbeeinflussungseinheit 2 weitergeleitet. Die manuelle Klangbeeinflussungseinheit 2 ist mit einer Bandbreitenauswerteeinheit 5 verbunden. Die Bandbreitenauswerteeinheit 5 führt zu einem Hüllkurvenmodulator 6. Der Hüllkurvenmodulator 6 weist eine automatische Auswahleinheit 7 auf und/oder arbeitet mit einer manuellen Auswahleinheit 8 zusammen. Die automatische Auswahleinheit 7 erhält als Fahrzeugparameter beispielsweise die Fahrzeuggeschwindigkeit v. Die manuelle Auswahleinheit 8 ist beispielsweise mit zwei Bedienelementen 9 und 10 versehen. Das Bedienelement 9 dient zur Auswahl der Wachheitsförderung, das Bedienelement 10 dient zur Auswahl der Entspannungsförderung.

In Abhängigkeit von den Ausgangssignalen der Auswahleinheiten 7 und/oder 8 sowie in Abhängigkeit von der in der Bandbreitenauswerteeinheit 5 ermittelten Bandbreite wird eine Hüllkurvenmodulation mit entsprechender Modulationsfrequenz und/oder mit entsprechendem Modulationshub vorgenommen. Hierzu kann im Hüllkurvenmodulator 6 beispielsweise eine Auswahl von verschiedenen Hüllkurven A, B, C und D abgelegt sein.

Das Ausgangssignal des Hüllkurvenmodulators 6 kann beispielsweise über eine automatische Klangbeeinflussungseinheit 3 an einen Lautsprecher 4 im Fahrzeug-Innenraum weitergegeben werden. Die manuelle Klangbeeinflussungseinheit 2 und die automatische Klangbeeinflussungseinheit 3 sind für die Erfindung nicht zwingend erforderlich und sind bereits aus dem Stand der Technik bekannt. Dabei kann über die manuelle Klangbeeinflussungseinheit 2 vom Kunden beispielsweise der Pegel bestimmter Frequenzbereiehe verstärkt werden. Mittels der automatischen Klangbeeinflussungseinheit 3 kann eine weitere Beeinflussung des Audiosignals in Abhängigkeit von verschiedenen Fahrzeugparametern vorgenommen werden. Bei Vorrichtungen zur Beeinflussung eines Audiosignals in einem Kraftfahrzeug nach dem Stand der Technik sind bisher lediglich die Komponenten 1, 2, 3 und 4 bekannt. Die Erfindung besteht im wesentlichen in der Zwischenschaltung der Komponenten 5, 6 zwischen den Klangbeeinflussungseinheiten 2 und 3.

Die Bandbreitenauswerteeinheit 5 kann die Bandbreite des vom Radio 1 ausgesendeten Audiosignais beispielsweise in zwei Kategorien einteilen: zum Beispiel Kategorie K1 für große Brandbreiten und Kategorie K2 für schmale Bandbreiten. Wird die Bandbreitenkategorie K1 erkannt, wählt der Hüllkurvenmodulator 6 einen großen Modulationshub H1 entsprechend der Hüllkurven A oder B aus. Wird die Bandbreitenkategorie K2 erkannt, wird vom Hüllkurvenmodulator 6 der niedrige Modulationshub H2 entsprechend der Hüllkurven C oder D gewählt.

Schließt die manuelle Auswahleinheit 8 durch die Betätigung des Bedienelements 9 auf die Auswahl der Wachheitsförderung, wird eine Modulation entsprechend der Hüllkurven B oder D mit einer hohen Modulationsfrequenz f2, insbesondere zwischen 10 und 20 Hz, vorgenommen. Dieselbe Hüllkurvenmodulation wird eingestellt, wenn die automatische Auswahleinheit 7 feststellt, daß die Fahrzeuggeschwindigkeit v beispielsweise oberhalb einer vorgegebenen Schwelle (z. B. 5 km/h) liegt.

Bei der Betätigung des Bedienelements 10 wird dagegen eine Modulation entsprechend der Hüllkurven A oder C mit einer niedrigen Modulationsfrequenz f1, f1 < 10 Hz, eingestellt, um die Entspannung zu fördern. Beispielsweise kann als Bedienelement die Taste 9 entfallen und lediglich die Taste 10 vorgesehen werden, so daß grundsätzlich die Wachheit gefördert wird und nur bei willkürlicher Betätigung der Entspannungstaste 10 die der Wachheit entgegenwirkende, also entspannende Frequenz ausgewählt wird. Die Betätigung des Bedienelements 10 ist beispielsweise bei einer Fahrpause auf dem Rastplatz sinnvoll.

Zur Auswahl der Hüllkurvenmodulation sind entsprechend dem angegebenen Beispiel demnach vier Hüllkurven A bis D möglich:
- Bandbreite K1 und Entspannung: ⇒ A
- Bandbreite K1 und Wachheit: ⇒ B
- Bandbreite K2 und Entspannung: ⇒ C
- Bandbreite K2 und Wachheit: ⇒ D

Ergänzend wird darauf hingewiesen, daß auch kontinuierlich veränderbare Modulationshübe und Modulationsfrequenzen möglich sind. Hierzu müßte eine komplexere Bandbreitenauswerteeinheit 5 und ein komplexerer Hüllkurvenmodulator 6 vorgesehen sein.

## Patentansprüche

1. Vorrichtung zur Beeinflussung eines Audiosignais in einem Kraftfahrzeug mit einem Hüllkurvenmodulator (6), der das Audiosignal mit einer Hüllkurve (A, B, C, D) moduliert, die eine die Wachheit des Fahrzeuginsassen beeinflussende Frequenz aufweist.

2. Vorrichtung nach Patentanspruch 1, dadurch gekennzeichnet, daß eine manuelle Auswahleinheit (8) vorgesehen ist, durch die mindestens eine Hüllkurve (B, D) mit einer die Wachheit fördernden Frequenz (f2) und mindestens eine Hüllkurve (A, C) mit einer der Wachheit entgegenwirkenden (entspannenden) Frequenz (f1) auswählbar ist.

3. Vorrichtung nach Patentanspruch 1 oder 2, dadurch gekennzeichnet, daß eine automatische Auswahleinheit (7) vorgesehen ist, durch die in Abhängigkeit von mindestens einem Fahrzeugparameter (v) mindestens eine Hüllkurve (B, D) mit einer die Wachheit fördernden Frequenz (f2) und mindestens eine Hüllkurve (A, C) mit einer der Wachheit entgegenwirkenden (entspannenden) Frequenz (f1) auswählbar ist.

4. Vorrichtung nach einem der Patentansprüche 1 bis 3, dadurch gekennzeichnet, daß eine Bandbreitenauswerteeinheit (5) vorgesehen ist, durch die die Bandbreite (K1, K2) des Audiosignals bestimmt wird, und daß der Pegel der Hüllkurvenschwingung (Modulationshub H1, H2) je höher eingestellt wird, um so größer die Bandbreite des Audiosignals ist.
